# EUROPEAN PATENT APPLICATION

(11) **EP 4 841 641 A1**
(43) Date of publication of application: **18.11.2026**
(21) Application number: 25177067.3
(22) Date of filing: 16.05.2025
(51) Int. Cl.: G16H 10/60, A61B 5/00, G06F 16/901, G06N 3/02, G06N 5/00, G06T 7/00, G16H 15/00, G16H 30/20, G16H 30/40, G16H 40/60, G16H 50/20, G16H 50/70, G16H 70/60

(54) **METHODS AND SYSTEMS FOR OUTPUTTING A PATHOLOGICAL LOCATION**

(71) Applicant: Siemens Healthineers AG, 91301 Forchheim (DE)
(72) Inventor: YU, Daphne, Yardley, 19067 (US); VARADAN, Yamini, East Brunswick, 08816 (US)
(74) Representative: Siemens Healthineers Patent Attorneys

(57) **Abstract**

A Computer-implemented method outputting a pathological location is provided. The method comprises a plurality of steps. One step is directed to receiving patient data. Another step is directed to applying a trained machine learning model to the patient data to determine one or more findings. Another step is directed to mapping the one or more findings to nodes of a clinical knowledge database to determine one or more mapped findings. The nodes of the clinical knowledge database comprise reference findings and/or reference data. Another step is directed to determining the pathological location associated with the one or more mapped findings. Another step is directed to outputting the pathological location.

## Description

Embodiments of the present invention relate to systems and methods for outputting a pathological location of a finding. In particular, embodiments of the present invention relate to the usage of trained machine learning models for medical data processing, and, in particular, for medical image data processing.

Detecting pathologies in a medical dataset is a widely applied, critical but complex and therefor often error prone task within medical data processing diagnosis workflows. Especially, automatically localizing the approximate pathological area would be of value for downstream medical diagnosis workflows.

Machine learning methods and algorithms are applied to medical data to generate insights and/or anatomic/pathological findings. Typically, such methods and algorithms can be run and/or trained on a computing unit. In general, machine learning methods have proven very versatile in various fields of application. They are used, for instance, to support decision making in autonomous driving. Likewise, machine learning methods are relied on for providing medical diagnoses by automated systems processing medical data such as medical images, patient data and/or reports.

However, due to the complexity and number of pathologies occurring in medical data, it is often not possible to extract the sought after pathological location in an efficient manner when applying machine learning methods to medical data. Furthermore, it is difficult to amass pathological data for training of machine learning model sufficiently. In many cases the inherent sensitivity of patient related data raises important patient privacy concerns.

One way of addressing this problem is to implement machine learning methods to detect organs and/or parts of organs within medical datasets. Here, the gross appearance of the organs is usually consistent enough to be detected even in the presence of pathologies.

However, one issue in this regard is that detecting the location of organs or parts of organs is not sufficient in differentiating clinical findings located at pathological areas. For example, highlighting to a patient his/her kidneys to explain signs of swelling and inflammation of tissues at a nearby portion of the ureter is not precise enough in a medical diagnosis process.

Known methods may allow for rough estimate of locations of pathological findings. The methods may be suitable to navigate to nearby neighborhood of a given description of a finding to speed up human interactions within the workflow. However, methods still lack the precision needed for full automated workflow for outputting the pathological location of a finding.

It is therefore an object of embodiments of the invention to provide improved pathological location detection methods and systems for supporting medical diagnosis workflows. In particular, it is an object of embodiments of the present invention to provide systems and methods which allow for a more efficient outputting of pathological locations of anatomical findings.

This object is solved by a computer-implemented method, an apparatus for implementing the computer-implemented method, corresponding computer-program products, and computer-readable storage media according to the main claims. Alternative and/or preferred embodiments are object of the dependent claims.

In the following, the technical solution according to the present invention is described with respect to the claimed apparatuses as well as with respect to the claimed methods. Features, advantages, or alternative embodiments described herein can likewise be assigned to other claimed objects and vice versa. In other words, claims addressing the inventive method can be improved by features described or claimed with respect to the apparatuses. In this case, e.g., functional features of the method are embodied by objective units or elements of the apparatus.

According to an aspect, a computer-implemented method for outputting a pathological location is provided. The method comprises a plurality of steps. One step is directed to receiving and/or obtaining patient data. Another step is directed to applying a trained machine learning model to the patient data to determine one or more findings. Another step is directed to mapping the one or more findings to nodes of a clinical knowledge database to determine one or more mapped findings. The nodes of the clinical knowledge database comprise reference findings and/or reference data. Another step is directed to determining the pathological location associated with the one or more mapped findings, in particular based on the reference findings and/or reference data. Another step is directed to outputting the pathological location.

Preferably, a trained machine learning model for determining one or more findings in a patient dataset may be provided. The trained machine learning model may be received, in particular at a processing unit, in particular from a remote unit, for example a server. Providing the trained machine learning model may comprise creating local version and/or update of the machine learning model, in particular at a processing unit. Providing the trained machine learning model may comprise providing a log file, wherein the log file comprises a parameterization of the trained machine learning model.

In general, a machine learning model is configured to provide a desired or predetermined kind of output by processing a certain kind of input data. Thereby, a machine-learning model mimics cognitive functions that humans associate with other human minds. In particular, by training based on training data, the machine learning model is able to adapt to new circumstances and to detect and extrapolate patterns. Other expressions for machine learning model may be machine learned model, trained function, trained machine learning model, trained mapping specification, mapping specification with trained parameters, function with trained parameters, algorithm based on artificial intelligence, or machine learned algorithm.

In general, parameters of a machine learning model can be adapted by training so as to obtain a model update. In particular, supervised training, semi-supervised training, unsupervised training, reinforcement learning and/or active learning can be used. Furthermore, representation learning can be used. In particular, the parameters of the machine-learned model can be adapted iteratively by several steps of training.

In particular, the machine learning model can comprise a neural network, a support vector machine, a decision tree and/or a Bayesian network, and/or the trained function can be based on k-means clustering, Q-learning, genetic algorithms, a transformer network and/or association rules. In particular, a neural network can be a deep neural network, a convolutional neural network or a convolutional deep neural network. Furthermore, a neural network can be an adversarial network, a deep adversarial network and/or a generative adversarial network. Moreover, a neural network can comprise a transformer network.

The machine learning model may in particular be any suitable language model. In one embodiment, the language model may be a custom and/or specifically trained language model for natural language processing, such as, e.g., BERT (Bidirectional Encoder Representations from Transformers). In another embodiment, the language model may be a pre-trained deep learning based LLM (large language model). The LLM may be based on the transformer architecture, which uses a self-attention mechanism to capture long-range dependencies in text. One example of a transformer-based architecture is GPT (generative pre-training transformer), which has a multilayer transformer decoder architecture that may be pretrained to optimize the next token prediction task and then fine-tuned with labelled data for various downstream tasks. GPT-based LLMs may be trained using reinforcement learning with human feedback for performing various natural language processing tasks.

The pathological location may refer to a specific area within an anatomical structure within a human body. An anatomical structure may comprise one or more organs of the patient (e.g., lung of a patient, or liver of a patient) or areas in between organs of the patient (e.g., muscles). In other words, pathological locations may be anatomical structures such as organs, or parts of an organs. Pathological locations may refer to areas where a pathology or abnormality is detected in a medical image. For example, a pathological location may be a kidney, a portion of the ureter and/or a specific part of the kidney showing signs of swelling and inflammation of tissue as an abnormality.

The patient data (also patient dataset) may be associated with one specific patient. In other words, patient data may differentiate from patient to patient. The patient data may comprise medical information and/or data about the patient. The patient data may include medical image data. The medical image data may be stored in a standard image format such as the Digital Imaging and Communications in Medicine (DICOM) format. The medical image data may be stored in a memory or computer storage system at a local site such as a Picture Archiving and Communication System (PACS). Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

According to some examples, the patient data may comprise at least one, preferably a plurality of medical image data sets, respectively showing a body part of a patient. Accordingly, the depicted body part of the patient in general comprises a plurality of anatomies and/or organs (also compartments or anatomic structures). As an example, the patient dataset may be or may include a chest image. The chest image may show lung tissue, bones, e.g. the rib cage, heart and aorta, lymph nodes and others. However, patient specific medical image data may also be received separately from the patient data.

According to some examples, the patient data may comprise at least one medical report, in particular radiology report. The medical report may for example comprise a textual description of information related to the patient. The description may include information on a body part, a finding, a diagnosis, a treatment and/or any other medical information regarding the patient.

The medical report may be in structured or un-structured form. A structured medical report facilitates communication of patient information. However, the presented methods are also capable of processing unstructured reports. The medical report may include at least one of the following sections:
Patient information section: This section includes the patient's demographic details, such as name, age, gender, and contact information.

Chief complaint section: Here, the patient's main reason for seeking medical attention is documented.

Past medical history section: This includes information about the patient's previous illnesses, surgeries, medications, and allergies.

Physical examination findings section: A thorough description of the patient's physical appearance, vital signs, and examination findings should be included.

Laboratory and diagnostic test results section: This section presents the results of any relevant tests or investigations conducted on the patient.

Assessment and diagnosis (sometimes also called impression) section: The healthcare professional's evaluation and diagnosis of the patient's condition are outlined here.

Treatment plan section: This section details the recommended treatment options, including medications, procedures, and lifestyle modifications.

Prognosis and follow-up section: The healthcare professional outlines the expected outcome of the patient's condition and provides instructions for follow-up care.

Alternatively or in addition, the patient data may comprise a two-dimensional pathology image data set, i.e., a so-called whole-slide image, depicting a tissue section of a patient.

According to some examples, the patient data comprises an image processing result associated to at least one medical image. In particular, the image processing result may be determined based on the medical image. The image processing result may be or may include, in particular, a (verified) detection result of a medical finding in a respective medical image, a (verified) classification result of a medical finding in a respective medical image, and/or a segmentation result of the respective medical image.

According to some examples, the patient data comprises information about the patient such as height, weight, age and/or medical history data of the patient, e.g. an appendix surgery.

A (medical) finding may indicate a certain condition or pathology of the patient. The condition or pathology may be relevant for the diagnosis of the patient. A finding may comprise a description of an anatomical site and/or a description of other aspects of the medical finding. The finding may be a data object comprising multiple data set and/or information associated with one or more medical findings. The (medical) finding may include an associated laterality and/or additional contextual site descriptions. The finding may also be referred to as clinical finding description phase. A finding may comprise a contextual laterality and/or site description describing a pathological location of the finding. The finding may comprise a pathological location.

A medical finding and/or anatomical site description may relate to an anatomical structure that differentiates the patient from other patients. Medical findings may be located within different organs of the patient (e.g., within the lung of a patient, or within the liver of a patient) or in between the organs of the patient. In particular, a medical finding may also relate to a foreign body.

In particular, a (medical) finding may relate to a neoplasm (also denoted as "tumor"), in particular, a benign neoplasm, an in-situ neoplasm, a malignant neoplasm and/or a neoplasm of uncertain/unknown behavior. In particular, a medical finding may relate to a nodule, in particular, a lung nodule. In particular, a medical finding may relate to a lesion, in particular, a lung lesion.

The (medical) finding may comprise a classification. The classification may relate to identify a finding type and or to provide a classification according to a plurality of predefined classes such as benign or malignant.

According to some examples, a mapped finding refers to a finding that has been associated with one or more nodes in the clinical knowledge database. For this, a reference finding and/or reference data linked to the node may be mapped to the finding. Mapping a reference finding and/or reference data (or data in general) to a finding may comprise adding information and/or data to the finding and/or amending information and/or data of the finding. The mapped finding may comprise the reference finding and/or reference data and the finding. The mapped finding may be related to or based on the reference finding and/or reference data and the finding. The mapped finding may be stored in a temporary storage device. The finding, in particular the information defining the finding, may be adapted with the reference findings and/or reference data yielding a mapped finding. In other words, by mapping the findings to the nodes of the clinical knowledge database the structured information in the database provides contextual and concentrated data to the findings.

In one example, the clinical knowledge database (also clinical database) is a large clinical knowledge Al embedding database. The clinical knowledge database may include a clinical knowledge ontology. Medical features and/or information are mapped to nodes of clinical knowledge ontology. For this a graph model and/or a language model may be used to provide the clinical knowledge database. The clinical knowledge database may include nodes to model clinical nomenclature and/or ontology.

The clinical knowledge database may be based on standard ontology graphs, such as, e.g., RadLex, UMLS (unified medical language system), or any other suitable imaging ontology.

Optionally, a graph model may be created to store relationships between nodes and edges of the clinical knowledge database to allow for more indirect description associations. For generating embeddings or vector representation of medical features, embedding models (e.g., text-ada-embedding-002 or even fine-tuned BERT) are used. In the graph model, the links between ontology concepts may be stored as embeddings (e.g., MR concept is a child of Diagnostic Imaging).

Optionally, in a language model, medical features and DICOM (digital imaging and communications in medicine) tags of an image (e.g., modality, study date, view position, pixel array, study description) and their link to the ontology concepts (e.g., RadLex identifiers) are converted to a paragraph description and stored as embeddings.

These two types of embeddings (from the language model and graph model) can then be combined to find a closest matching one to a request.

In one embodiment, the extracted features are associated with the corresponding nodes based further on metadata, which may be non-standardized and potentially site specific. For example, the extracted features may comprise image acquisition using contrast with an MR (magnetic resonance) protocol. The image acquisition label and the associated MR acquisition parameters are then associated with the corresponding node for subsequent retrieval.

Medical features may be associated with one or more corresponding nodes of the clinical knowledge database. The nodes may in particular be represented as language embedded vectors of the descriptions and aliases of medical features and/or findings.

According to some examples, reference findings and/or reference data in the clinical knowledge database refer to standardized and/or structured medical information. The reference findings and/or data may be structured within nodes of the clinical knowledge database, in particular representing anatomical objects of interest, pathological locations and/or specific medical conditions.

Reference findings in particular may be predefined medical conditions and/or pathologies classified and/or categorized within the database. In other words, reference findings may serve as benchmarks or standards against which patient-specific findings can be compared. For example, a reference finding might include detailed descriptions of various types of tumors, their typical locations, and associated symptoms.

Reference data in particular may include additional contextual information that might support the determining of the pathological location of findings. Reference data can encompass a wide range of medical knowledge, such as anatomical descriptions, typical imaging characteristics, and known correlations between different medical conditions. In other words, reference data may provide background information and context of the findings. For example, reference data may comprise a laterality and/or site description of a finding and/or condition.

Obtaining and/or Receiving patient data may comprise receiving stored data from a memory or computer storage system, e.g. a Picture Archiving and Communication System (PACS). Further patient data may be received, for example, directly from an image acquisition device, e.g. recorded medical images. The patient data may be received by loading patient specific medical data and/or information from a storage or memory of a computer system, or by receiving a patient dataset from a remote computer system.

Applying a trained machine learning model to the patient data to determine one or more findings may comprise applying a pre-trained machine learning model to a patient-specific dataset to analyze the dataset. Analyzing the dataset may comprise extracting features from medical images, reports or other relevant medical information comprised in the dataset. For example, the patient data may comprise a radiology report. The trained machine learning model may be applied to the radiology report that includes textual descriptions and/or phrases of medical findings and/or diagnosis information. The machine learning model may process the report to identify and/or extract textual descriptions and/or phrases as relevant findings. The relevant findings may be prioritized. The relevant findings may be outputted, e.g. as a (prioritized) list.

As another example, the trained machine learning model might analyze the radiology report and extract phrases indicating the presence of abnormalities such as "lung nodule," "swelling in the kidney," or "inflammation of the liver." These extracted findings are then prioritized based on their clinical significance and presented in a list format for further review by medical professionals.

Mapping the one or more findings to nodes of a clinical knowledge database to determine one or more mapped findings may comprise determining one or more nodes of a clinical knowledge database that match the finding determined from the patient data. With inputting the findings as request into the database, the matching nodes may be determined that are associated with the same or similar reference findings. Optionally, the matching nodes may be ranked. The nodes may present anatomical objects of interest that may be mapped to the findings. If a matching node is found, the associated information and/or pathological location are mapped to the findings resulting in a mapped finding. Mapping in general in this context may refer to adding an information of the database to a finding and/or to amending a finding based on the information of the database.

Determining the pathological location associated with the one or more mapped findings may comprise extracting and/or determining the pathological location from the data included in and/or associated with the mapped findings. For this, the reference findings and/or reference data extracted from the clinical knowledge database may be used. For each finding a pathological location may be determined.

For example, the mapped findings may comprise a plurality of - optionally ranked - possible and/or related pathological locations. Determining the pathological location may comprise determine the pathological location most likely associated with one finding. The most likely pathological location may be determined with a score, for example.

Outputting the pathological location may comprise displaying the pathological location on a device, such as a computer screen, and/or providing the pathological location for methods and/or data processing devices. The pathological location may for example be visualized in a medical image by highlighting a specific area within an anatomical structure associated with the finding. Additionally or alternatively, the pathological location can be output by including, in particular formatting, the pathological location into a report and/or data format for other medical systems, e.g. for further comprehensive analysis and/or recording methods.

Alternatively or in addition, outputting the pathological location may include providing the pathological location to a downstream process, for example for a downstream image analysis process. In particular, a image processing algorithm may be selected based on the pathological location. For example, there may be different algorithms specialized on analyzing different regions of the body and the pathological location may be used as a decision aid for selecting the most appropriate algorithm. Alternatively or in addition, an image from a plurality of images may be selected and displayed based on the determined pathological location. Alternatively or in addition, a rendering algorithm may be executed based on the determined pathological location. For example, the field of view and/or the angle of view of the rendering algorithm may be set based on the determined pathological location.

The method provides a significant advantage by enabling precise localization of pathological location of findings derived from patient data, in particular unstructured patient data. The method may allow for synergistically combining available medical data to provide an automatic workflow for outputting pathological locations. The context of a medical knowledge database may be leveraged to include and ground relevant medical information associated with the findings, including contextual details such as laterality and site descriptions. The method advantageously improves an automated medical diagnosis workflow outputting a relevant pathological location. This may allow for a more efficient diagnosis workflow in medical environments. Further, the method may also reduce the amount of data which is needed to determine a pathological location.

For training of the models there are various options which are further elaborated below. For example, supervised learning may be applied. This method involves training the model on a labeled dataset, where each input is paired with a correct output. The model learns to map inputs to outputs by minimizing the difference between its predictions and the actual labels. The advantage is the high accuracy and reliability when a large, high-quality labeled dataset is available.

Alternatively or in addition, unsupervised learning may be used. In this approach, the model is trained on an unlabeled dataset. The model learns to identify patterns and structures in the data without explicit instructions on what to look for. The model can leverage vast amounts of unlabeled data, which is more readily available than labeled data. The learning data can include high amounts of unlabeled text data from sources like books, articles and/or websites. The data may be collected from the internet without specific labels.

According to some examples, the machine learning model may be trained by applying training datasets to the machine learning model to determine parameters of the machine learning model and/or to parametrize the machine learning model.

According to some examples, the machine learning model may be updated by updating one or more parameters of the machine-learned model. According to some examples, updating may be performed by applying a further training dataset to the machine learning model and/or if an evaluation result, in particular by determined by a user, indicates an increased error rate of the machine learning model. With that the machine learning model can be successively optimized.

A machine learning model (also "trained function") learns by adjusting parameters that determine the mapping from input data to output data. In neural networks, these parameters are, for example, the weights or weighting parameters (e.g., edge weights) of individual layers and nodes. In the process of supervised learning of the trainable function, the backpropagation method can be used. During training, the trained function is applied to training input data to generate corresponding output values, whose target values are known in the form of training output data. The difference between the output values and the training output data can be used to introduce a cost or loss functional as a measure of how well or poorly the trained function performs the given task. The goal of training is to find a (local) minimum of the cost functional by iteratively adjusting the parameters (e.g., edge weights) of the trained function. This optimization problem can be carried out using a stochastic gradient descent method or other approaches known in the field.

According to embodiments, training datasets would include medical training datasets and training comparison datasets, as well as, depending on the configuration of the trained function, associated verified reference datasets and/or verified (particularly relative) similarity measures. The verified reference datasets could be based on a user's annotation made based on an analysis or diagnosis of the medical training dataset. The same applies to the verified similarity measures.

Training the trained function could, according to some embodiments, thus include applying the trained function to the medical training datasets or training comparison datasets to generate output values and comparing the output values with verified reference datasets and/or verified similarity measures. Based on the comparison, one or more parameters of the trained function can then be adjusted.

For example, the medical training datasets can include patient data. The verified reference datasets can include findings. In other words, the medical training datasets can be used as input data for the trained function. The verified reference datasets can be used as comparison data for the output data of the trained function. In other words, the training data can include input data, patient data particularly comprising a report for example, with associated expected findings.

According to some examples, the clinical knowledge database is generated by receiving one or more medical images, extracting features from the one or more medical images using one or more machine learning models, associating the extracted features with corresponding nodes of the clinical knowledge database, and outputting the clinical knowledge database with the extracted features associated with the corresponding nodes.

The nodes of the clinical knowledge database may be output with the extracted features associated with the corresponding nodes. For example, the clinical knowledge database can be output by storing the clinical knowledge ontological database on a memory or storage of a computer system or by transmitting the clinical knowledge ontological database to a remote computer system and/or server.

According to an embodiment, the computer-implemented method comprises a plurality of further steps. One step is directed to receiving one or more patient specific images associated with and/or included in the patient data. Another step is directed to determining one or more landmarks within the patient specific images. Another step is directed to matching the one or more landmarks to the one or more mapped findings such that the mapped findings comprise one or more matched landmarks.

The patient data may comprise at least a medical/radiological report. The patient data may comprise historical and/or previous medical images of a patient. The patient specific images may in particular relate to the latest, or current medical image associated with the patient. The mapped findings may comprise one or more matched landmarks.

According to some examples, the one or more patient specific images (also short "image") comprise a plurality of medical images, medical image data and/or medical image datasets.

The patient specific image may comprise a figurative description of an anatomical object of interest within the patient's body. In general, a medical image may depict any captured anatomical object, such as, e.g., organs, bones, vessels, abnormalities, etc. Medical images may be recorded with any suitable modality and/or image acquisition device, such as, e.g., CT (computed tomography), MRI (magnetic resonance imaging), US (ultrasound), x-ray, or any other medical imaging modality or combinations of medical imaging modalities. Medical images may be 2D (two dimensional) images, 3D (three dimensional) volumes, or 4D (four dimensional) volumes (3D plus time).

The medical image dataset may relate to a medical image study. The medical image dataset may relate to three-dimensional data sets providing three dimensions in space or two dimensions in space and one dimension in time, to two-dimensional data sets providing two dimensions in space, and/or to four-dimensional data sets providing three dimensions in space and one dimension in time.

The medical image dataset may depict a body part of a patient in the sense that it contains three-dimensional image data of the patient's body part. The medical image dataset may be representative of an image volume. The patient's body part may be comprised in the image volume.

The medical image dataset comprises image data, for example, in the form of a two- or three-dimensional array of pixels or voxels. Such arrays of pixels or voxels may be representative of intensity, absorption or other parameters as a function of three-dimensional position, and may, for example, be obtained by suitable processing of measurement signals obtained by a medical imaging modality.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system), an ultrasound imaging system or the like. Specifically, computed tomography is a widely used imaging method and makes use of "hard" X-rays produced and detected by a specially rotating instrument. The resulting attenuation data (also referred to as raw data) is presented by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas and the signals are analyzed by a computer creating detailed images of the internal structures in any section of the human body.

The medical image dataset may comprise a plurality of images or image slices. The slices may respectively show a cross-sectional view of the image volume. The slices may comprise a two-dimensional array of pixels or voxels as image data. The arrangement of slices in the medical image data set may be determined by the imaging modality or by any post-processing scheme used.

Further, the medical image dataset may be a two-dimensional pathology image data set, i.e., a so-called whole-slide image, depicting a tissue section of a patient.

According to some examples, the medical image data set may have been acquired at a local site. The local site may be a medical institution and/or hospital, in particular comprising at least one medical imaging modality.

The medical image data set may be stored in a standard image format such as the Digital Imaging and Communications in Medicine (DICOM) format and in a memory or computer storage system at the local site such as a Picture Archiving and Communication System (PACS). Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

According to some examples, the one or more patient specific images comprise a local data set and/or parameterization data, in particular associated with the patient data.

According to some examples the local data set comprises, for each medical image data, a verified image processing result, in particular, a verified detection result of a medical finding in the respective medical image data, a verified classification result of a medical finding in the respective medical image data, and/or a segmentation of the respective medical image data.

According to some examples, the parameterization data comprises the verified image processing result or a parameterization thereof. As the case may be, the parameterization may comprise an indication of the medical imaging modality (or modalities) with which the medical image data has been acquired, imaging parameters used upon acquiring the medical image data, a medical finding comprised in the medical image data, a segmentation of an object comprised in the medical image data.

Landmarks may refer to specific anatomical points and/or regions within the patient specific images. The landmarks can include various segmented organs, anatomical structures and human body parts. The landmark may comprise a point, in particular described by coordinates in relation to the patient specific image, and/or a region, in particular described by an edge and/or boarder of a region of the patient specific image. In particular, the landmark may comprise an image coordinate node.

The one or more patient specific images may be received and/or obtained, for example, directly from an image acquisition device, as medical images are recorded as the one or more patient specific images at the image acquisition device. The one or more patient specific images may be received by loading previously acquired medical images from a storage or memory of a computer system, or by receiving medical images of a specific patient from a remote computer system.

The landmarks may be detected by applying a segmentation algorithm to the patient specific images. The segmentation algorithm processes the patient specific images to identify one or more landmarks. The segmentation algorithm may analyze the patient specific images for predefined set of detectable landmarks for a consistent identification of anatomical structures across different medical images. The landmarks may comprise coordinates indicating and/or referring to anatomical structures in a medical image. In particular, the landmarks may be indicative for a pathological location.

Matching the one or more landmarks to the one or more mapped findings may comprise comparing location information, for example coordinates, of the mapped findings to the landmarks.

Within mapping, the coordinate of the landmarks may be associated with the findings. The highest-ranking matching landmarks may be determined that are associated with the patient specific images. The determined findings may be matched to landmarks representing detected anatomical structures in the patient specific images. If a match is found, the associated coordinates - a location information within the patient specific image - are used to connect the location within the patient specific image to the finding. The coordinates may comprise spatial information (e.g., coordinates, paths, models, etc.). In one embodiment, the coordinates may comprise converted 3D coordinates to a specific view, e.g. a reformatting, clip plane, or surface view from a plurality of coordinates.

By correlating the location information of the landmarks detected in patient specific medical images to the findings, the pathological location can accurately be determined. Further, the spatial coordinates of pathological locations in the images are associated with the findings for future analysis steps and/or methods.

According to some examples, the one or more landmarks matched to the one or more mapped findings may be output. Outputting the landmarks may comprise outputting the patient specific images. Outputting the landmarks may comprise adapting the patient specific images to only comprise the landmarks, e.g. by highlighting the landmarks in the image.

According to some examples, the landmarks are determined by applying a trained machine learning model, in particular feature encoder, to the patient specific images. According to some examples, the landmarks comprise encoded features as identified by the machine learning model based on a predefined set of landmarks. According to some examples, the machine learning model is different and/or independent from the trained machine learning model used to determine the findings. This may mean that the machine learning model has been trained independently from the trained machine learning model used to determine the findings and/or comprises a different architecture.

According to some examples, a segmentation may be directed to an organ, finding, or other compartment of a body part of the patient. According to some examples, the step of segmenting may mean obtaining an outline of the respective organ or compartment and/or delineating the organ or compartment from the rest of the image data to determine one or more landmarks of the image. The landmark in particular may comprise a delineated organ or compartment and/or may be located on an outline of a respective organ or compartment.

For a review on transformer networks used a machine learning model, in particular a feature encoder, reference is made to Vaswani et al., "Attention Is All You Need", in arXiv: 1706.03762, June 12, 2017, the contents of which are herein included by reference in their entirety.

According to some examples, off-the-shelf generative Al functions may be used such as DALL-E or Midjourney. According to other examples, customized generative Al functions based on a transformer architecture may be used which are trained based on pairs of parameterizations and corresponding (real) data.

According to an embodiment, the computer-implemented method comprises a plurality of further steps. One step is directed to receiving one or more atlases comprising a plurality of reference landmarks. Another step is directed to matching the one or more mapped findings to the reference landmarks such that the mapped findings comprise one or more matched reference landmarks.

The mapped findings may comprise one or more matched reference landmarks. Matching a reference landmark to the mapped finding may comprise adding information and/or data of the reference landmark to the mapped finding and/or amending information and/or data of the mapped finding to include a link to the reference landmark.

The atlas may be a canonical human atlas and/or graph model comprising a spatial-ontological relation between a target pathological location and nearby detected and/or possible locations, labeled anatomical landmarks. The atlas may comprise a human model. In other words, the atlas represents a reference patient and/or human model. The atlas may be generated and/or received from a database and/or source. An example for an atlas is the Human Reference Atlas available from HuBMAP. Each atlas is typically pre-annotated with a densely sampled set of spatial (anatomical) landmarks matching those in established ontology concepts.

Anatomical landmarks, also referred to as reference landmarks, may be landmarks comprised by an atlas. Each anatomical landmark may consist of a 3D coordinate relative to the human model of the atlas. Anatomical landmarks may refer to specific anatomical points and/or regions within the human model of the atlas. Anatomical landmarks can include various segmented organs, anatomical structures and human body parts. Anatomical landmarks may comprise a point, in particular described by a node in a graph model of the atlas and/or a region, in particular described by coordinates in relation to the human model of the atlas. The graph model may represent the connections of the nodes. In particular, the landmark may comprise densely sampled set of spatial anatomical landmarks matching those in ontology concepts.

The atlas may be received out of a plurality, for example from a database and/or server. The one or more atlases may be received by loading a, in particular previously generated, atlas from a storage or memory of a computer system, or by receiving an atlas form a remote storage device, such as a cloud storage. For example, the Human Reference Atlas may be received from HuBMAP. HuBMAP, or the Human BioMolecular Atlas Program, is a project providing a comprehensive atlas of the human body at the molecular level. This publicly available atlas can be used to map various biological structures and their functions within the human body.

The matching the one or more mapped findings to the anatomical landmarks of the received atlas is in particular only performed if a pathological location is not determinable from one of the detectable landmarks in the patient specific image. This may be in particular the case since the detectable landmarks in the patient specific image typically only cover sparse sampling of gross anatomy features such as parts of an organ and not a pathology. In other words, the atlas is utilized to find a nearest matched landmark within a human atlas.

For matching the one or more mapped findings to the anatomical landmarks more than one atlas can be used, such as atlases for male and female or pediatrics separately. As a sub step, matching the one or more mapped findings to the anatomical landmarks in the atlas may comprise a graph-based search, where edges of the graph model are connections within a medical ontology representing anatomical spatial-ontological relationships. As another sub step, the closest anatomical landmark is determined based on related structures given by a node within that graph model.

For example, the right kidney anatomical region is a node in the graph where, upper and lower parts, or the parenchyma of the kidney are related nodes within the graph model. Therefore, by performing a graph search the nearest anatomical landmark is determined within different levels of related nodes, in particular body structure and/or regions, to match the mapped findings.

Utilizing an atlas advantageously may enable a precise localization of pathological findings and provide context information for determining pathological locations. Additionally, an atlas supports the combination of various medical data sources and information.

According to some examples, an atlas is received and/or chosen out of a plurality of atlases based on the patient data.

According to an embodiment, the computer-implemented method comprises a plurality of further steps. One step is directed to receiving one or more patient specific images associated with the patient data. Another step can be directed to receiving one or more atlas comprising a plurality of reference landmarks. Another step can be directed to determining anchor landmarks in the patient specific images based on the reference landmarks. Another step can be directed to correlating mapped findings to anchor landmarks within the patient specific images such that the mapped findings comprise one or more matched anchor landmarks.

The mapped findings may comprise one or more matched anchor landmarks. Matching an anchor landmark to the mapped finding may comprise adding information and/or data of the anchor landmark to the mapped finding and/or amending information and/or data of the mapped finding to include a link to the anchor landmark.

An anchor landmark represents in particular a landmark which is detected and/or determined in the atlas and the specific patient image. In other words, the anchor landmark is a shared landmark from both atlas and patient specific image.

The one or more patient specific images may be received, for example, directly from an image acquisition device, as medical images are recorded as the one or more patient specific images at the image acquisition device. The one or more patient specific images may be received by loading previously acquired medical images from a storage or memory of a computer system, or by receiving medical images of a specific patient from a remote computer system.

The atlas may be received out of a plurality, for example from a database and/or server. The one or more atlases may be received by loading a, in particular previously generated, atlas from a storage or memory of a computer system, or by receiving an atlas form a remote storage device, such as a cloud storage.

Determining anchor landmarks in the patient specific images based on the reference landmarks may comprise comparing the reference landmarks of the atlas to the detectable landmarks in the patient specific image. A matching of the reference and/or anatomical landmarks of the atlas with the image may comprise a graph-based search, where edges of the graph model are mapped to the unstructured landmarks of the patient specific image.

In addition, the mapped findings may be correlated to anchor landmarks within the patient specific images in another step. The step may comprise estimating a pathological location of the finding based on the (shared) anchor landmark present in the atlas and patient specific image representing a spatial relationship between the finding and anchor landmark.

Advantageously this may provide a relationship between the finding and anchor landmarks from the atlas. The relationship can be leveraged to estimate the target pathological location based on the anchor landmark in the patient specific images.

According to an embodiment, the landmarks and/or anchor landmarks in the patient specific images are determined on at least one of imaging modality, acquisition parameter, acquisition orientation, image appearance, anatomical field of view, or classifications and detections.

By determining the landmarks and/or anchor landmarks in the patient specific images on at least one factor such as imaging modality, acquisition parameter, acquisition orientation, image appearance, anatomical field of view, or classifications and detections, it allows for an adaptable and precise identification of landmarks. By considering those various factors the detected landmarks are aligned with the specific characteristics of the medical images. This enhances the adaptability and reliability of the landmark detection process, leading to more accurate localization of pathological locations.

According to an embodiment, the mapping of the one or more findings to nodes of a clinical knowledge database comprises a plurality of steps. One step is directed to parsing the patient data and/or the findings into one or more vectors. Another step is directed to performing a vector search between the vectors representing the patient data and vectors representing nodes of the clinical knowledge database to determine a similarity between the one or more vectors and the nodes of the clinical knowledge database.

A vector in particular refers to a numerical representation of patient data. Various attributes such as medical history and or a report may be converted into numerical form and organized, for example, into an array or list of numbers. The nodes of the clinical knowledge database may comprise a vector representing the data of the node or associated with the node. The vectors representing nodes of the clinical knowledge database may comprise the data and information of one node or associated with one node in a numerical form.

Parsing the patient data and/or the findings into one or more vectors may comprise converting patient data into numerical form and/or organizing it into vectors. Parsing the patient data into one or more vectors may comprise transforming information into number arrays and/or a list. For example, laterality and/or site information might be encoded into numerical values using transformer or encoder.

The vector search may comprise comparing the vectors representing the patient data with the vectors representing the nodes of the clinical knowledge database to find similarities. In other words, it is measured how close and/or similar the vectors are in a multi-dimensional space. Performing the vector search may comprise determining a cosine similarity, Euclidean distance, or other metrics that quantify the similarity between vectors. By performing the vector search, the nodes in the clinical knowledge database are determined match to the patient data, in particular with consideration of a comparison or similarity metric, e.g. a score, prioritization scheme and/or threshold.

The vector representation enables comparison of the patient data with other vectors representing nodes in a clinical knowledge database. By performing a vector similarity search, a similarity between the patient data vectors and the clinical knowledge vectors and/or nodes can be determined to identify relevant medical conditions and/or data associated with the findings.

According to an embodiment, the determining a pathological location of the one or more mapped findings comprises at least one of:
- filtering of location pointer data associated with the one or more mapped findings based on body structure laterality and/or site,
- prioritizing the location pointer data to determine a first location pointer,
- matching at least the first location pointer of the mapped findings to pathological locations associated with nodes of the clinical knowledge database to determine an approximated target location as pathological location.

Location pointer data refers to specific data points for identifying and/or localizing anatomical structures and/or pathological findings within the human body. In other words, location pointer data is data for accurately mapping findings to specific pathological locations. Location pointer data may comprise reference points within the human body. Reference points represent points that are well-defined and universally recognized in medical practice, for example body structure laterality or site information.

Body structure and/or laterality is an information indicating whether a finding is on the left or right side of the human body. Site information are specific details about an exact anatomical location, such as an organ or tissue.

One step comprises filtering the location pointer data associated with one or more mapped findings based on body structure laterality and/or site. The step may comprise examining the location pointer data to determine whether the findings are on the left and/or right side of the body and/or to identify the specific anatomical location. For example, if a lesion is found in the left hippocampus, the location pointer data is filtered to ensure that only information about the left side of the brain and the hippocampal region is considered in the matching step. In other words, by filtering the location pointer data the location pointer data is narrowed down to the most pertinent information.

Another step is to prioritize the filtered location pointer data to determine a first location pointer. Prioritize may comprise ranking the filtered location pointer data based on its relevance and importance to the specific anatomical location. For instance, if multiple data points are available for the left hippocampus, the most relevant and accurate data point is chosen as the first location pointer by estimating a distance to the finding data.

Another step comprises matching at least the first location pointer of the mapped findings to pathological locations associated with nodes of the clinical knowledge database to determine an approximated target location as the pathological location. The filtered and/or prioritized location pointer is compared with reference data in a clinical knowledge database. For example, the first location pointer for a lesion in the left hippocampus is matched with similar pathological locations in the database to determine the most accurate target location.

Matching location pointer data to the mapped findings may improve localizing of pathological locations of the findings by ensuring that the data is filtered and prioritized based on body structure laterality and specific anatomical sites.

According to an embodiment, mapping the one or more findings to nodes of a clinical knowledge database comprises verifying the findings and/or ranking the findings based on the clinical knowledge database.

Verifying the findings by mapping one or more findings to nodes of a clinical knowledge database may comprise comparing determined findings to reference data in the database to identify findings that might be wrong and/or not relevant. A plurality of findings may be determined by applying the trained machine learning model to the patient data. Verifying the findings may comprise narrowing down the plurality of findings to exclude finding. The trained machine learning model might for example determine wrong, not relevant and/or an abundance of findings. Verifying the findings may comprise discarding and/or confirming finings on basis of reference data of the database.

Ranking findings based on the clinical knowledge database may comprise prioritizing the, in particular verified, findings to determine a relevance score. For example, the findings are ranked according to their similarity to the reference data in the clinical knowledge database. For instance, if multiple data points are available for a finding, is chosen as the primary finding. Another prioritization can also be determined based on the scores of data point of the database associated with the findings.

By mapping the one or more findings to nodes of a clinical knowledge database by verifying the findings and/or ranking the findings advantageously the data of findings might be enhanced or incorrect or irrelevant information discharged.

According to an embodiment, applying the machine learning model to the patient data to determining one or more findings comprises determining a section of the patient data to which the trained neural network is applied to.

According to an embodiment, the patient data comprises a radiological report. The determining of a section of the patient data in particular comprises determining a section of the radiological report.

The determining of a section of the patient data may be dependent on a local site. The local site may, for instance, relate to an organization in which the computer-implemented method is to be deployed. In particular, the local site may relate to healthcare environments or organizations or facilities such as hospitals, laboratories, practices, universities or associations of one or more of the aforesaid.

A section in patient data can refer to various specific portions or segments of the patient data. Sections can comprise medical image data, such as two-dimensional or three-dimensional arrays of pixels or voxels representing different body parts, like a chest image showing lung tissue and the rib cage. Sections can comprise radiology reports or parts of radiology reports, e. g. textual descriptions about body parts, findings, diagnoses, and treatments. Additionally, sections can comprise pathology image data, e.g. whole-slide images that depict tissue sections. By focusing on these specific sections, healthcare professionals can apply trained neural networks more effectively to analyze and interpret the data, leading to more accurate and efficient diagnoses and treatment plans.

In other words, advantageously by focusing on specific sections, the trained machine learning model can be applied effectively to analyze the patient data.

According to an embodiment, outputting the pathological locations comprises outputting the pathological locations in the patient specific images on a display device.

According to an embodiment, outputting the pathological locations comprises providing the pathological locations for a data processing method.

Outputting the pathological locations on a display device may comprise displaying the medical images and the associated pathological locations on an output device, such as a monitor or computer display. Outputting the pathological locations on the display device may comprise rendering the medical image and pathological locations according to a computed appearance and then transmitting the rendered data as graphical signal to the display device.

Providing the pathological locations for a data processing method comprises providing the pathological locations via a file, in particular a log file. Additionally, the pathological locations can be formatted into a report and/or data format. Providing the pathological locations may comprise generating a file or report comprising coordinates and/or descriptions of the pathological locations. The file or report can be integrated and/or stored into electronic health records (EHRs).

Outputting pathological locations in patient-specific images on a display device and/or outputting pathological locations for data processing methods may improve the diagnostic workflows.

According to some examples, a log file may comprise the pathological locations. According to some examples, the log file is formatted according to the JSON standard. JSON stands for JavaScript Object Notation and is an open standard file format and data interchange format that uses human-readable text to store and transmit data objects consisting of attribute-value pairs and arrays. With that, the interoperability of the method can be improved.

According to an embodiment, outputting the pathological location comprises multiple steps:
- receiving viewing preferences for medical images,
- generating a display layout for medical images fortified with the pathological locations based on the viewing preferences,
- outputting the display layout.

The viewing preferences for medical images may be defined, for example, based on at least one of anatomical display orientation, windowing, rendering mode, and/or any other suitable viewing preferences.

A display layout may in particular comprise information and/or settings for an output device, in particular to display the medical images. The display layout of the one or more medical images is automatically generated based on the viewing preferences. A language model may transform the viewing preferences to internal viewing implementations and setting of an output device, e.g. a display. The display layout system may provide a translation of user input of instructions in clinical nomenclature to related medical images.

The viewing preferences may in particular be received via a user input. The user input may comprise a description of medical images and/or viewing preferences for the medical images. The viewing preferences may be extracted from textual/voice commands by a language model parser. The user input may comprise a language prompt for viewing preferences. The viewing preferences may be derived from the language prompt. The viewing preferences may be sent as language prompt to a output device and/or interface unit to translate the viewing preferences to rendering parameters. The language prompt may be a prompt or input to a language model defining a specific rendering input schema. For example, the language prompt may indicate to language model that renderer can support viewing the patient in the anterior, left, and right orientations and instruct language model to translate the user input viewing preferences to one of these orientations.

A display layout of the one or more medical images may be automatically generated based on the viewing preferences. The display layout may comprise rendering parameters. In one example, the viewing preferences extracted from textual/voice commands by a language model parser are sent to a language model as a language prompt to translate the viewing preferences to rendering parameters. A renderer may generate display layout according to the rendering parameters.

A renderer comprised in the output device may generates display layout according to the rendering parameters. For example, the display layout can be output by displaying the display layout on a display device of a computer system, by storing display layout on a memory or storage of a computer system, or by transmitting the display layout to a remote computer system Embodiments described herein may be implemented in, e.g., PACS (picture archiving and communication system) viewer applications. For example, when a user loads patient data in a PACS viewer application, the user can provide user input in a chat window and the system may automatically select medical images and generate the display layout for displaying the medical images to the user.

By receiving viewing preferences from a user and outputting the image data according to a display layout, an efficient system for displaying the pathological locations associated with the findings can be provided.

According to some examples, the patient specific images can be fortified to display the pathological locations. Fortifying the patient specific images may comprise overlaying and/or highlighting specific regions where abnormalities or pathological findings are determined. By fortifying the patient specific images, the illustration of the localization of a pathology may be improved for clinicians.

According to an aspect, an apparatus is provided. The apparatus comprises means for receiving patient data. The apparatus comprises means for applying a trained machine learning model to the patient data to determine one or more findings. The apparatus comprises means for mapping the one or more findings to nodes of a clinical knowledge database to determine one or more mapped findings. The apparatus comprises means for determining a pathological location associated with the one or more mapped findings. The apparatus comprises means for outputting the pathological location.

The means for receiving patient data may comprise a temporary storage unit. A temporary storage unit in general is a designated space and/or container within a storage and/or computing unit, configured to store data items or information for a defined period of time. After the defined period of time before the data items or information may be moved to permanent storage location, processed further and/or discarded. For example can the temporary storage unit store the received patient data for up to 48 hours before deleting the data.

The means for applying a trained machine learning model to the patient data, the means for mapping the one or more findings to nodes of a clinical knowledge database and/or the means for determining a pathological location associated with the one or more mapped findings can comprise and/or implemented by a computing unit.

The computing unit and/or processing unit may be a data processing system or part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories, and combinations thereof. The one or more memories may store instructions for carrying out the method steps according to the invention. The computing unit may comprise the temporary storage unit. The hardware can be configurable by the software and/or be operable by the software. Generally, all units, sub-units or modules may at least temporarily be in data exchange with each other, e.g., via a network connection or respective interfaces. Consequently, individual units may be located apart from each other. Further, the computing unit may be configured as an edge device.

The means for outputting the pathological location may comprise a interface unit. The interface unit may comprise an interface for data exchange with one or more local clients, e.g., via the Internet. The interface unit may be further adapted to interface with one or more users of the system, e.g., by displaying the result of the processing to the user (e.g., in a graphical user interface).

The apparatus may in particular be configured to execute one or more steps of the methods as herein described in their various aspects and/or examples.

The advantages described in connection with the method aspects and examples may also be realized by the correspondingly configured apparatus' components.

According to some examples, the apparatus may further comprise means for providing the clinical database and/or the machine learning model. For example, means for providing the clinical database may comprise a database unit and/or a cloud storage. Means for providing the machine learning model may comprise a storage unit, a server unit and/or a cloud storage.

According to another aspect, the present invention is directed to a computer program product comprising instructions which, when the program is executed by a computer, cause the computer to carry out at least one step of the method according to one or more of the above method aspects and examples. In other words: The present invention is directed to a computer program product comprising program elements which induce a computing unit to perform the steps according to one or more of the above method aspects and examples, when the program elements are loaded into a memory of the computing unit.

According to another aspect, the present invention is directed to a computer-readable medium comprising instructions which, when executed by a computer, cause the computer to carry out at least one step of the method according to one or more of the above method aspects and examples. In other words: The present invention is directed to a computer-readable medium on which program elements are stored that are readable and executable by a computing unit to perform the steps according to one or more method aspects and examples, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale.

In the following:
FIG. 1 schematically depicts a computer-implemented method for outputting a pathological location according to an embodiment;
FIG. 2 schematically depicts a computer-implemented method for outputting a pathological location according to an embodiment;
FIG. 3 schematically depicts a computer-implemented method for outputting a pathological location according to an embodiment;
FIG. 4 schematically depicts an exemplary data flow diagram in connection with a computer-implemented method for outputting a pathological location according to an embodiment;
FIG. 5 schematically depicts an exemplary data flow diagram in connection with a computer-implemented method for outputting a pathological location according to an embodiment;
FIG. 6 schematically depicts an embodiment of an apparatus according to an embodiment.

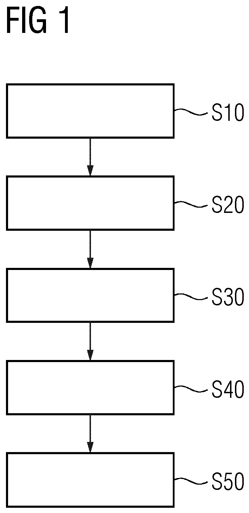

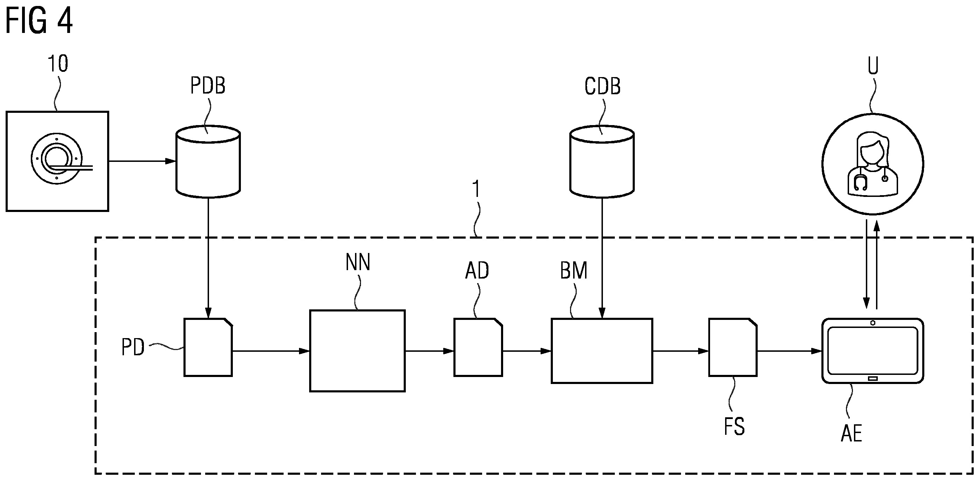

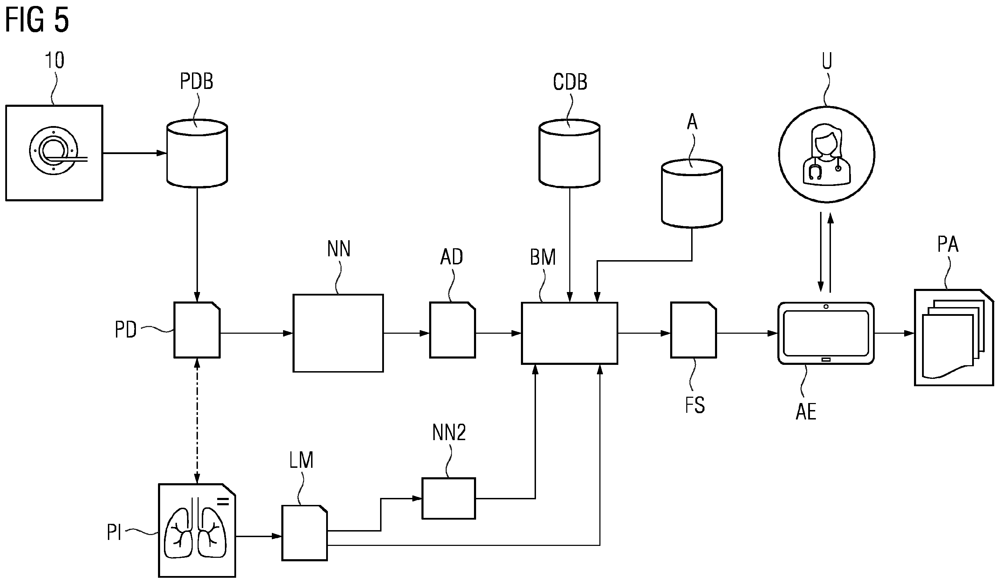

Figure 1, 2 and 3 depicts a computer-implemented method for outputting a pathological location. Corresponding data streams are illustrated in Figure 4 and 5. The method comprises several steps. The order of the steps does not necessarily correspond to the numbering of the steps but may also vary between different embodiments of the present invention. Further, individual steps or a sequence of steps may be repeated. According to methods of the present inventions, the steps may be executed separately.

At step S10, a patient data PD is received, in particular at the apparatus 1. According to embodiments, the patient data PD is received from a patient database PDB. Specifically, the patient database PDB may be configured to store and/or process any medical data of patients, in particular the patient data PD. According to embodiments, the patient data PD is recorded, at least partially at a medical system 10. The patient data PD can be received directly from the medical system 10. Specifically, the medical system 10 may be configured to record medical image data of a patient.

According to some examples, the patient data PD comprises medical image data, in particular a patient specific image PI. According to some examples, the patient data PD comprises at least one medical report, in particular a radiological report.

At step S20, a machine learning model NN may be applied to the patient data PD- The machine learning model NN may be received at the apparatus 1. The machine learning model NN may be provided to and administrated by the apparatus 1, in particular a computing unit BM of the apparatus 1.

The machine learning model NN may be readily trained and meant to be deployed at the apparatus 1 according to the learned task. According to embodiments, this learnt task may comprise an automated processing of patient data PD for deriving (medical) findings AD. Specifically, the machine learning model may be configured to process a medical report of a patient in order to determine medical findings. In particular, the machine learning model NN may be a language processing model to extract relevant finding information out of a text and/or section of the medical report.

In particular, the machine learning model NN may generate a textual summary of the patient data PD as a finding. For instance, if the patient data PD includes image data visions transformers may be used which have been trained to analyze medical image data and consolidate the findings into textual impressions (like the ones to be found in medical reports).

According to some examples, the medical report may contain information about parts of the patient's torso and findings comprising lesions in the lung or liver of the patient. The machine learning model NN, by applying it to the medical report, may determine the findings AD, in particular the lesions in the lung or liver of the patient.

The machine learning model NN may be further trained based on the local data. This leads to a (local) update NN' of the machine learning model NN. According to some examples, such training may happen on the fly, e.g., when a user reviews the processing results of the machine learning model NN. For instance, a radiologist may decline or accept findings AD provided by the machine learning model NN. Further, the radiologist may add findings AD not determined by the machine learning model NN. A user U inputs may be used as ground truth for further optimizing, that is, training the machine learning model NN. The ground truth together with the underlying local data may form the training data of the machine learning model NN.

Optionally, the machine learning model NN may function on the basis of a natural language prompt. The prompt may be seen as an instruction or control command for the machine learning model NN. Such prompt may be generated in an optional sub-step on the basis of the patient data. In a way step S20 may be seen as a translation step of translating the patient data PD into a set of findings AD on which the other steps of the method be based upon and/or on the basis of which other functions and/or machine learning models may take action.

At step S30, the one or more findings AD are mapped to nodes of a clinical knowledge database CDB to determine one or more mapped findings. The nodes comprise reference findings and/or reference data. In other words, the findings AD are compared to the reference findings and/or reference data of the clinical knowledge database CDB represented as nodes. The mapping of findings AD to nodes of the database may comprise determining of reference findings and/or reference data which correspond to the findings AD. In other words, reference findings and/or reference data is determined which is medically relevant for the determined findings AD. For example, findings AD comprising lesions in the liver of the patient may be compared to and/or mapped to reference findings and/or reference data comprising liver lesions and/or liver cancer.

The mapping of findings AD to nodes of the database CDB may comprise adding at least part of the determined reference findings and/or reference data to the findings AD to received mapped findings. The reference findings and/or reference data may, in particular contain pathological information, in particular pathological locations FS.

In particular, may the reference findings and/or reference data be used for validating the findings AD determined by the machine learning model NN. The findings AD may be amended, modified and/or edited based on the reference findings. The information of the reference findings may be added to the information of the findings AD.

At step S40, the pathological location FS associated with the one or more mapped findings is determined. The determining of the pathological location FS associated with the one or more mapped findings may comprise extracting a pathological location information or description out of the information and/or data associated with the one or more mapped findings. In other words, the data and/or information of the mapped findings may be searched for a pathological location information. In case a pathological location information cannot be determining the pathological location FS may comprise determining the next closest pathological location. Determining the next closest pathological location may comprise determining a body region and/or part adjacent to the one of the findings AD. The steps S20 and S30 may be repeated for the at least one body region and/or part adjacent to the one of the findings AD.

Finally, at step S50, the pathological location FS may be output. In particular, the pathological location FS may be output to one or more other (medical) devices. In particular, the pathological location FS may be output to a user U, in particular via a display device AE. In particular, the pathological location FS may be output to provide (training) data to updated machine learning model NN.

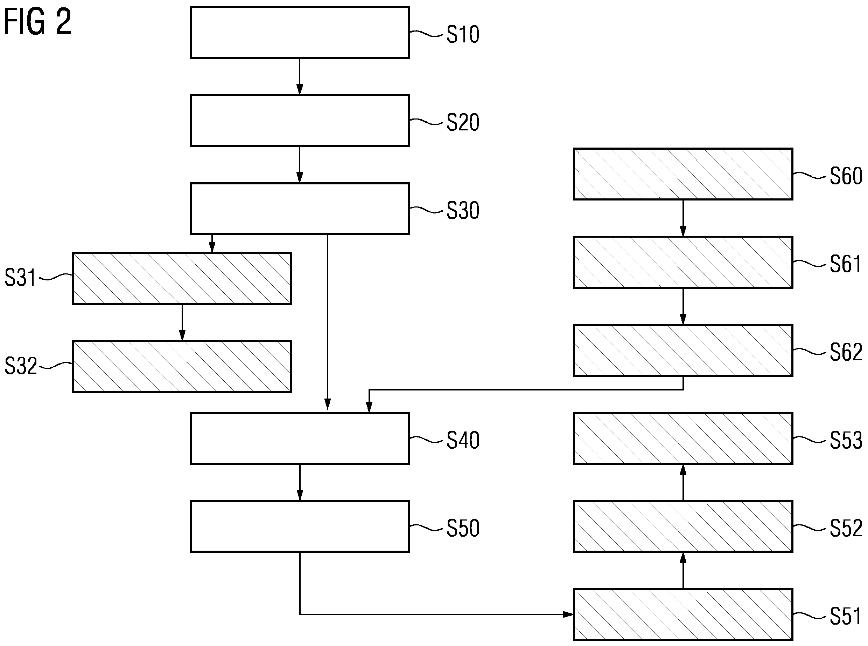

Coming to Figure 2, at step S60, one or more patient specific images PI associated with or included in the patient data may be received. The patient data received in step S10 may in particular be a radiological report and/or contain historic/previous medical images, in which case the with the report associated (current and/or latest) medical images PI may be received and/or retrieved from the patient data PD.

For example, the patient data PD may comprise a report about lesions in the lung or liver of a patient. The associated patient specific image PI may show parts of the patient's torso comprising the lung or liver of the patient.

According to other examples, the patient specific image PI may comprise digital pathology images of the patient. The findings AD may relate to a segmentation of the digital pathology image according to one or more tissue types.

At step S61, one or more landmarks LM within the patient specific images PI are determined. The landmarks LM may for example be determined with a segmentation model. The determining of landmarks LM may comprise identifying specific anatomical points and/or regions within the patient specific images PI. In other words, the determined landmarks LM can include various segmented organs, anatomical structures and human body parts and/or regions.

At step S62, the one or more landmarks LM are matched to the one or more mapped findings. The mapped findings may comprise one or more matched landmarks LM. The mapped findings may be amended, modified and/or edited based on the landmarks LM. The information of the landmarks LM may be added to the information of the mapped findings.

The landmarks LM determined in the patient specific images PI and/or the location information and/or data comprised in the determined landmarks LM may be compared to landmarks LM and/or location information and/or data comprised in and/or associated with the mapped findings.

In case a match is determined the landmark LM, in particular the location information and/or data, may be added to the mapped findings.

At Step 51, viewing preferences for medical images, in particular the patient specific images PI, may be received, in particular from a user U. A user input may be received at an interface device AE. The viewing preferences may be determined based on the user input.

In one embodiment, the viewing preferences are defined based on at least one of anatomical display orientation, windowing, or rendering mode of the patient specific images.

At step S52, a display layout for medical images, in particular the patient specific images PI, is generated. The medical images may be fortified with the pathological locations based on the viewing preferences.

At step S53, the display layout is output, in particular to a user U. In particular, a fortified patient specific image comprising the pathological locations FS may be output via a display device to a user.

At step S31, the patient data PD is parsed into one or more vectors. On the basis of the one or more vectors the database CDB may be searched for similar data. In other words, the one or more vectors may present a search string to the database CDB.

At step S32, a vector search between the vectors representing the patient data PD and vectors representing nodes of the clinical knowledge database CDB is performed. A similarity between the one or more vectors and the nodes of the clinical knowledge database CDB is determined.

In other words, a similar vector search in the clinical knowledge database CDB is performed. A similar vector search may comprise comparing the vectors representing the patient data PD with the vectors representing the nodes of the clinical knowledge database CDB to find similarities. Essentially, it is measured how close and/or similar the vectors are in a multi-dimensional space.

The vector search may for example involve determining a cosine similarity, Euclidean distance, or other metrics that quantify the similarity between vectors. By performing this vector search, the nodes in the clinical knowledge database can be identified that match the patient data, considering a comparison or similarity metric, such as a score, prioritization scheme, or threshold.

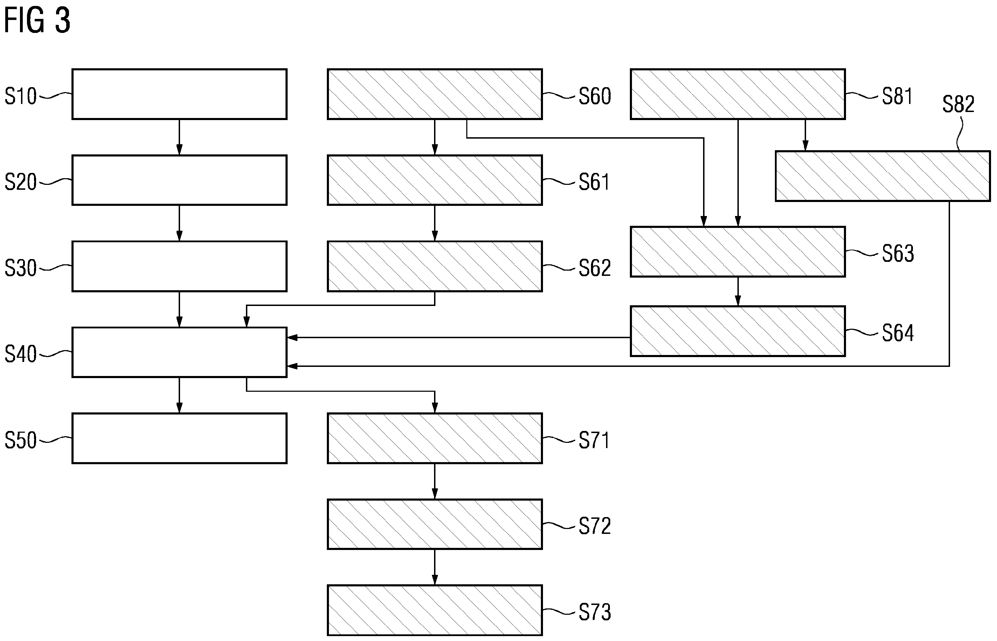

Turning to Fig. 3, at step S63, anchor landmarks in the patient specific images PI are determined based on reference landmarks from an atlas.

The anchor landmarks may be determined by comparing the reference landmarks from the atlas to the detectable landmarks within the patient specific images PI. The determining of the anchor landmarks may involve a graph-based comparison method, where the edges of a graph model are mapped to the unstructured landmarks of the patient-specific image.

By identifying these anchor landmarks, a spatial relationship can be established between the findings and the anatomical structures within the patient-specific image PI, in particular without a present, to the findings matching landmark.

At step S64 the mapped findings are correlated to the anchor landmarks determined within the patient specific image PI. One or more matched anchor landmarks may be added to the mapped findings. In other words, the mapped findings may comprise the matched anchor landmarks.

The pathological location FS of the finding AD is estimated based on the shared anchor landmarks present in both the atlas and the patient-specific images PI. With the spatial relationship between the finding AD and the anchor landmark, a target pathological location FS can be accurately determined without a directly matching landmark LM in the patient specific image PI.

The step may be implemented by a machine learning model to output a result of the correlation of the findings to the anchor landmarks. The machine learning model be configured to analyze patterns and/or relationships within the findings and the anchor landmarks to determine the correlation result.

At step S81, one or more atlases comprising a plurality of reference landmarks is received.

The atlases may serve as comprehensive graphs and/or maps that include detailed anatomical reference landmarks. The atlas can be sourced from databases A or servers. The atlas may be pre-generated and/or stored in a memory or storage unit, e.g. a computing unit. The atlas may be retrieved and/or loaded from a memory or storage unit A to a processing unit BM.

As an example, the Human Reference Atlas could be received at the processing unit BM from HuBMAP at step S81.

That followed, at step S82, the one or more mapped findings are matched to the reference landmarks of the received atlas. Step S82 may particularly be performed if a pathological location FS is not determinable from the detectable landmarks in the patient-specific image PI. Particularly in this case, the atlas may be utilized to find the nearest matched reference landmark within a human model and/or graph of the atlas. The matching may comprise using multiple atlases, such as those for different genders or age groups, and/or may include a graph-based search. The edges of the graph model of the atlas represent anatomical spatial-ontological relationships in particular. By performing a graph search, the closest anatomical reference landmark is determined within different levels of related nodes.

At step S71, location pointer data associated with the one or more mapped findings is filtered based on body structure laterality and/or site. The location pointer data may comprise information and/or data about a body structure laterality and/or site of one or more findings. The mapped findings may be analyzed to determine whether the findings are on the left or right side of the body and/or to identifying a specific anatomical region within the body.

For example, if a lesion is found in the left hippocampus, the location pointer data is filtered to ensure that only information about the left side of the brain and the hippocampal region is considered in the matching step. In other words, the filtering narrows down the data to the information, which allows for accurately localizing pathological findings.

At step S72, the location pointer data is prioritized to determine a first location pointer. In particular, if one or more location pointer is filtered out in step S71, the filtered location pointers may be ranked based on relevance sources. The relevance source may be determined by a trained function. Step S72 may also be a sub step of S71 and/or fully comprise by S71.

For instance, if multiple location pointer are available for a particular anatomical site or region, the most relevant and accurate location pointer may have a high relevance source. If a finding is associated with this location pointer, the location pointer is chosen as the first location pointer for estimating a target pathological location.

At step S73, at least the first location pointer of the mapped findings is matched to the mapped findings to determine an approximated target location as pathological location FS. The mapped findings may comprise pathological locations associated with nodes of the clinical knowledge database CDB. These pathological locations FS of the mapped findings can be checked with the first location pointer for a matching pathological location FS.

Step S73 may comprise comparing the filtered and prioritized location pointer data with reference data in the clinical knowledge database CDB associated with the findings. The most accurate target pathological location FS is identified for the findings. For example, if the first location pointer indicates a finding in the left hippocampus, this location pointer is matched with similar reference pathological locations of the database associated with the finding as mapped findings to determine the precise target pathological location FS.

Figures 4 and 5 depict data flow diagrams of embodiments of the method shown in Figures 1, 2 or 3. Data streams corresponding to embodiments of the method are illustrated. The method comprises several steps. The order of the steps does not necessarily correspond to the depicted data streams. Further, individual steps or a sequence of steps may be repeated.

Figure 4 shows a medical system 10 as a starting point of a data flow. Medical images captured by the medical system 10 may directly be provided to a machine learning model NN as patient data PD. A patient database PDB connected to the medical system 10 may also provide the patient data PD to the machine learning model NN. The medical images captured by the medical system 10 as well as associated medical information such as reports may be stored at the patient database PDB. The machine learning model NN may be applied to the patient data PD to determine the findings AD. The findings AD may be provided to a processing unit BM. The processing unit BM may be connected to a clinical database CDB. The processing unit BM may be configured to determine and/or output pathological locations FS associated with the findings AD. The pathological locations FS may be output by an interface device AE, in particular a display device, to an user U. The user U may provide an input to the interface device AE.

Figure 5 shows an additional image data flow. The patient specific image PI, that might be comprised in the patient data PD, is provided. The patient specific image PI contains landmarks LM. The landmarks LM might be provided to the processing unit BM additional to the findings AD. The processing unit BM is connected to the clinical database CDB and may additionally be connected to an Atlas database A. The landmarks LM might also be provided to a machine learning model NN2, which might be connected or comprised by the processing unit BM. The interface unit AE may be configured to output images PA containing the pathological locations FS.

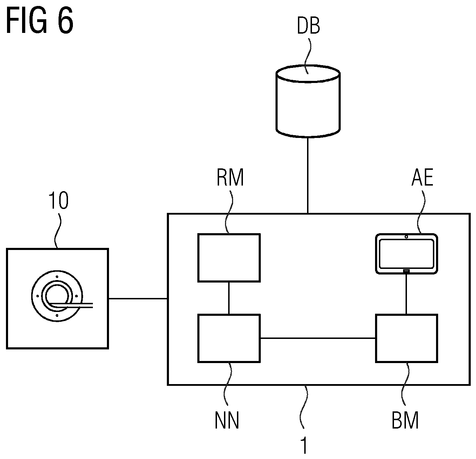

Figure 6 depicts an example apparatus 1 for outputting a pathological location FS. Apparatus 1 is adapted to perform the method according to one or more embodiments, e.g., as further described with reference to Figures 1 to 3.

Apparatus 1 comprises a temporary storage unit RM, processing unit BM and interface unit AE. The units may be interfaced via a network.

The apparatus 1 may be capable of creating, training, updating, and applying a machine-learned model NN. The machine learning model NN may generally be configured provide findings AD based on patient data PD. This may include detection of findings in medical image data, detection of findings in report data, the annotation of findings, e.g., in terms of orientations or landmark detection and the like.

The units RM, BM, AE of the apparatus 1 may be hosted on a server, which may be a cloud server or a local server. However, the units RM, BM, AE of the apparatus 1 also may be implemented using any other suitable computing device(s). The apparatus 1 may be connected to a database DB. The database DB may comprise a clinical knowledge database CDB, a patient database PDB and/or an atlas database A. The database DB might be configured for centrally storing training data for evaluating the findings AD of the machine learning model NN.

The apparatus 1 and/or the processing unit BM may be or comprise a computing unit. The computing unit may comprise one or more processors and a working storage. The one or more processor(s) may include, for example, one or more central processing units (CPUs), graphics processing units (GPUs), and/or other processing de-vices. The Computing unit may further comprise a micro-controller or an integrated circuit. Alternatively, computing unit may comprise a real or virtual group of computers like a so called 'cluster' or 'cloud'. The working storage may include one or more computer-readable media such as a RAM for temporally loading data. The working storage may further store information accessible by the one or more processors, for performing method steps according to one or more embodiments herein described. The working storage by also be or comprised by the temporary storage unit RM.

The interface unit AE can include any suitable components for interfacing with one or more networks, including, for example, transmitters, receivers, ports, controllers, or other suitable components.

The database DB may be realized as a cloud storage. Alternatively, the database DB may be realized as a local or spread storage, in particular, within the premises of the apparatus 1.

The database DB may be part of a hospital information systems (HIS), radiology information systems (RIS), clinical information systems (CIS), laboratory information systems (LIS) and/or cardiovascular information systems (CVIS), picture archiving and communicating system (PACS) or the like.

As an example, the data items, in particular patient data PD, of the database DB may relate to laboratory test results and/or pathological data and/or medical imaging data, electronic medical records, and any combination thereof.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention. Independent of the grammatical term usage, individuals with male, female or other gender identities are included within the term.

## Claims

1. A computer-implemented method for outputting a pathological location comprising:
receiving (S10) patient data,
applying (S20) a trained machine learning model to the patient data to determine one or more findings,
mapping (S30) the one or more findings to nodes of a clinical knowledge database to determine one or more mapped findings,
wherein the nodes comprise reference findings and/or reference data,
determining (S40) the pathological location associated with the one or more mapped findings, and
outputting (S50) the pathological location.

2. The computer-implemented method of claim 1, wherein the method further comprises:
receiving (S60) one or more patient specific images associated with the patient data, determining (S61) one or more landmarks within the patient specific images;
matching (S62) the one or more landmarks to the one or more mapped findings, such that the mapped findings comprise one or more matched landmarks.

3. The computer-implemented method of one of the previous claims, wherein the method further comprises:
receiving (S81) one or more atlases comprising a plurality of reference landmarks, matching (S82) the one or more mapped findings to the reference landmarks, such that the mapped findings comprise one or more matched reference landmarks.

4. The computer-implemented method of one of the previous claims, wherein the method further comprises:
receiving (S60) one or more patient specific images associated with the patient data, receiving (S81) one or more atlases comprising a plurality of reference landmarks, determining (S63) anchor landmarks in the patient specific images based on the reference landmarks,
correlating (S64) mapped findings to anchor landmarks within the patient specific images, such that the mapped findings comprise one or more matched anchor landmarks.

5. The computer-implemented method of any of the claim 2 or 4,
wherein the landmarks and/or anchor landmarks in the patient specific images are determined based on at least one of imaging modality, acquisition parameter, acquisition orientation, image appearance, anatomical field of view, or classifications and detections.

6. The computer-implemented method of one of the previous claims, wherein mapping the one or more findings to nodes of a clinical knowledge database comprises:
parsing (S31) the patient data into one or more vectors,
performing (S32) a vector search between the vectors representing the patient data and vectors representing nodes of the clinical knowledge database to determine a similarity between the one or more vectors and the nodes of the clinical knowledge database.

7. The computer-implemented method of one of the previous claims, wherein determining a pathological location associated with the one or more mapped findings comprises at least one of:
filtering (S71) of location pointer data associated with the one or more mapped findings based on body structure laterality and/or site,
prioritizing (S72) the filtered location pointer data to determine a first location pointer, matching (S73) at least the first location pointer of the mapped findings to pathological locations associated with nodes of the clinical knowledge database to determine an approximated target location as pathological location.

8. The computer-implemented method of one of the previous claims, wherein mapping the one or more findings to nodes of a clinical knowledge database comprises:
verifying the findings and/or
ranking the findings based on the clinical knowledge database.

9. The computer-implemented method of one of the previous claims, wherein applying the trained machine learning model to the patient data to determine one or more findings comprises:
determining a section of the patient data, in particular a radiological report comprised in the patient data, to which the trained neural network is applied to.

10. The computer-implemented method of one of the claims 2 to 9, wherein outputting the pathological location comprises outputting the pathological location in the patient specific images on a display device.

11. The computer-implemented method of one of the previous claims, wherein outputting the pathological location comprises providing the pathological location for a data processing method.

12. The computer-implemented method of one of the previous claims, wherein outputting the pathological location comprises:
receiving (S51) viewing preferences for medical images,
generating (S52) a display layout for medical images fortified with the pathological locations based on the viewing preferences,
outputting (S53) the display layout.

13. An apparatus comprising:
means for receiving patient data,
means for applying a trained machine learning model to the patient data to determine one or more findings,
means for mapping the one or more findings to nodes of a clinical knowledge database to determine one or more mapped findings,
means for determining a pathological location associated with the one or more mapped findings, and
means for outputting the pathological location.

14. Computer program product comprising instructions which, when the program is executed by a computer, cause the computer to carry out at least one step of the method according to any of claims 1 to 12.

15. Computer-readable medium comprising instructions which, when executed by a computer, cause the computer to carry out at least one step of the method according to any of claims 1 to 12.
